(19) Europäisches Patentamt
European Patent Office
Office européen des brevets

(11) **EP 3 238 716 A1**

(12) **EUROPEAN PATENT APPLICATION**
published in accordance with Art. 153(4) EPC

(43) Date of publication:
01.11.2017  Bulletin 2017/44

(21) Application number: 15871399.0

(22) Date of filing: 23.12.2015

(51) Int Cl.:
*A61K 9/51* (2006.01)   *A61K 31/722* (2006.01)
*A61K 31/51* (2006.01)   *C08B 37/08* (2006.01)
*C08L 5/08* (2006.01)

(86) International application number:
**PCT/CL2015/000067**

(87) International publication number:
**WO 2016/101081 (30.06.2016 Gazette 2016/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: 23.12.2014  CL 20143514

(71) Applicant: **Universidad De Concepcion
Concepción (CL)**

(72) Inventors:
• **VON PLESSING ROSSEL, Carlos Guillermo
Concepción (CL)**
• **CASTILLO QUIROGA, Eileen Valeska
Concepción (CL)**

(74) Representative: **Carvajal y Urquijo, Isabel et al
Clarke, Modet & Co.
Suero de Quiñones, 34-36
28002 Madrid (ES)**

(54) **NANOPARTICLES BASED ON CHITOSAN FOR THE TRANSPORT OF PEPTIDES WITH ACTIVITY IN THE CENTRAL NERVOUS SYSTEM**

(57)    The technology corresponds to nanoparticles based on chitosan for the transport of peptides with activity in the central nervous system, consisting of: (a) chitosan, as a base; (b) sodium triplyphosphate (TPP), as a cross-linking agent; and (c) thiamine pyrophosphate (TDP), as a signalling agent. These nanoparticles are used to produce a medicament that can be used for treating disorders of the central nervous system and can be administered intravenously or intranasally.

**Description**

**TECHNICAL SECTOR**

The technology described is destined for the pharmaceutical and health sector, since it includes the creation of a chitosan-based nanoparticle system for the transport of peptides with activity in the central nervous system. This technology can be used as an alternative treatment for a series of illnesses in which the neuroinflammatory cascade generates changes in the blood-brain barrier.

**PREVIOUS TECHNIQUE**

The blood-brain barrier (BBB) represents an obstacle to pharmacological treatments because it is difficult for drugs to pass through it to reach brain tissue. Even when administered intravenously, drugs attain very low concentrations in the central nervous system (CNS). This barrier selectively stops the passage of substances to the brain interstitium, with the result that many drugs that have proved useful in the treatment of systemic disorders are ineffectual against similar disorders in the CNS, due to their incapacity to cross the barrier: neuropeptides, proteins and anticancer drugs are important examples of therapeutic agents that have difficulty passing through the barrier. For this reason there are drugs on the market that, in order to reach acceptable concentrations in the brain, are administered in such high doses that they often cause severely adverse reactions in peripheral organs.

The tumour necrosis factor alpha (TNF- ) is a cytokine implicated in systemic inflammation and is a member of a group of cytokines that stimulate the acute phase reaction. It is mainly produced by activated macrophages, although it can also be produced by other types of cells. The principal function of TNF is the regulation of immune cells. TNF- , which is an endogenous pyrogen, can induce a fever, producing cell death through apoptosis, and induce sepsis (through IL1 and the production of IL-6), that in turn can induce cachexia and inflammation, and inhibit tumourigenesis and viral replication.

The deregulation of TNF production has been associated with a variety of human diseases, including Alzheimer's, in which neuronal damage caused by neurotoxic factors initiated by inflammatory reactions is related to cognitive and motor deterioration, which contributes to the rupture of the BBB, allowing the entry of leukocytes into the brain and thereby accelerating a neuroinflammatory cascade.

The TNF-$\alpha$ blocking molecule Etanercept (E1) is a soluble fusion protein that consists of 2 identical chains of TNF-R2 joined to the Fc domain of a human immunoglobulin IgG. Etanercept competitively inhibits TNF-$\alpha$, preventing it from binding with its membrane receptors. Etanercept is used to alleviate the symptoms of autoimmune disorders, such as rheumatoid arthritis, psoriatic arthritis, juvenile idiopathic arthritis, ankylosing spondylitis and chronic plaque psoriasis.

Of particular relevance are the results obtained after the perispinal administration of etanercept in patients with Alzheimer's disease, who have shown clear improvement in learning, memory and verbal fluency from the very first few minutes after the perispinal injection. The clinical effects suggest that this treatment approach, in addition to its usefulness for other forms of Alzheimer's disease, could be particularly useful for patients with frontotemporal dementia and a frontal variant of Alzheimer's. However, the means of administering the active principle is one of the limitations on its use, for which reason its possible incorporation into a nanoparticle system would help the administration of etanercept, reducing the associated risk for patients.

It can therefore be seen that it is important to regulate the presence of TNF-through control systems capable of reaching the CNS and controlling any brain damage, as is the case with nanoparticle systems.

Nanoparticle development is an alternative way to transport neuropeptides and drugs, since they are pharmaceutical formulations that can be directed to a target site and can protect the drug from physiological conditions that could alter its physicochemical properties, making it possible to reduce the doses and therefore some of the adverse effects. This type of transport can be used as a vehicle with the objective of increasing the bioavailability of molecules with a pharmacological effect in the CNS, increasing the selectivity for a blank and reducing the toxicity of the drug, among other uses.

The use of nanoparticle systems for the encapsulation of drugs can help the transport of the latter to the site of therapeutic action, increasing their average life and their controlled release over time. Additionally, because they are small particles they have a high surface area-volume relationship.

Nanoparticles in particular have aroused great interest as modified release systems for drugs, above all for non-oral administration methods, such as those that require an injection or need to be deposited on mucous surfaces such as that inside the nose. Nanoparticles are defined as particles whose size is smaller than $1\mu$m, this being one of the factors that most influence their internalisation in mucous and epithelial tissues, as well as their transport inside cells. The surface charge of nanoparticles determines their mucoadhesive properties and both characteristics can be controlled by changing certain conditions and variables of the obtention process.

One of the most important advantages of these systems is their capacity to cross biological barriers, a basic requirement for the use of these polymers in humans or animals being that they are biodegradable, biocompatible and non-toxic molecules for the biological environment. For this reason, a very limited number of molecules can be used to prepare biodegradable materials.

Chitosan is one of the biopolymers that is most studied for its use in modified release systems, as well as being one of the most abundant in nature. It is obtained from chitin, which forms part of the support structure of numerous living organisms, such as arthropods (crustaceans and insects), molluscs and fungi. Because of its cationic nature and its gelling and filmogenic properties, I chitosan is a vehicle for the encapsulation of drugs, protecting them and releasing them in a controlled way, in addition to aiding their absorption through the epithelium; at the same time, chitosan has the necessary properties for its use in the industry, namely, biodegradability, biocompatibility and low toxicity. In the last few years, the study of chitosan has focused above all on improving the release and absorption of the so-called therapeutic biomolecules, such as proteins and DNA, as well as drugs like corticoids, antipsychotics, etc.

The combination of all these characteristics of nanoparticles prepared from chitosan results in a desirable product for the pharmaceutical industry, both nationally and internationally. In Chile, the development of new pharmaceutical forms is an area of the pharmaceutical industry that has gained in importance over the years, due to the growth of the industry and the use of Chilean raw materials. The considerable increase in neurodegenerative diseases and the search for new treatments to control these diseases or restore the BBB mean that the implementation of a vehicle capable of generating appropriate treatment for these pathologies has become a matter of urgency and a growing focus of study in the field of nanomedicine.

Calvo *et al* (1997) described the preparation of nanoparticles from chitosan by ionic gelation of chitosan and sodium tripolyphosphate (TPP). These present a series of interesting characteristics that make them promising vehicles for the release of macromolecules, such as proteins and DNA. Some of these properties are their obtention through a process that does not require the incorporation of high quantities of energy or organic solvents, their adjustable size depending on the obtention parameters, the modulation of their positive charge, and their capacity to associate with peptides, proteins, oligonucleotides and plasmids.

TPP is a non-toxic agent (recognised as GRAS by the FDA in the US) that is able to form gels when it binds with chitosan through ionic interaction. The formulation of nanoparticles through ionic interaction between chitosan and TPP is relatively simple because it involves the mixing of two aqueous phases at ambient temperature without the use of organic solvents, the last-mentioned being a great advantage when scaling up processes to higher production levels, in comparison with other methods and polymers used to make nanoparticles.

Due to its cationic nature and its gelling and filmogenic properties, chitosan is a vehicle for the encapsulation of drugs, protecting them and releasing them in a controlled way, in addition to aiding their absorption through the epithelium. Chitosan can be degraded by enzymes such as; glucosamine-glucosamine hydrolase, glucosamine-N-acetyl-glucosamine hydrolase and N-acetyl-glucosamine-N-acetyl-glucosamine hydrolase. However, eight chitosans have been identified in humans, three of which have shown enzyme activity. These three forms are: acidic mammalian chitinase (AMCase), di-N-acetylchitobiase and chitotriosidase.

In neurodegenerative diseases such as Alzheimer's the BBB represents an obstacle for pharmacological treatments because it is difficult for drugs to pass through it to reach brain tissue. Even when administered intravenously, drugs remain at very low concentrations in the central nervous system (CNS). This barrier prevents the passage of substances to the brain interstitium in a selective way, so that many drugs that are useful in the treatment of systemic disorders are ineffectual against similar disorders of the CNS, because of their incapacity to cross this barrier.

The transport of substances through the BBB takes place in three ways: diffusion of lipophilic substances; specific transport of hydrosoluble substances through facilitated transport with or without energy consumption; and through ionic channels. At the same time there are also specific transporters for vitamins in the BBB, one example being thiamine, for which there are proteic transporters that allow its passage from the blood to the brain. The first transporter has high affinity and interacts with thiamine and acetylcholine (OCT-3), and the second transporter has low affinity and only interacts with thiamine (ThTR1).

Thiamine pyrophosphate (TPP) is used as a signal agent in the fabrication process of NPs because of its capacity to bind to the thiamine receptors in the BBB. It is a derivative of the vitamin B1, which plays an essential role as cofactor in key reactions of the metabolism of carbohydrates, is also involved in the metabolism of branched-chain amino acids, can play the role of coenzyme in nerve cells, and is of vital importance in the supply of energy to cells. Its main structural characteristic is the presence of phosphate groups, which give it the capacity to form salts with the amino groups of the chitosan, helping to increase chitosan's solubility in water.

Despite the afore-mentioned, it is still necessary to acquire technologies that make possible the effective transport to the CNS of substances or peptides that succeed in crossing the BBB without affecting its integrity.

**BRIEF DESCRIPTION OF THE FIGURES**

Figure 1: NPs of chitosan/TPP obtained through optimisation, analysed using TEM.
Figure 2: Optimisation of the production process of NPs of chitosan/TDP/TPP analysed using TEM.
Figure 3: Dynamic light scattering analysis (A). Suspension of NPs of chitosan/TPP. (B) Suspension of NPs of chitosan/TDP/TPP.
Figure 4: Zeta potential analysis (A) Suspension of NPs of chitosan/TPP. (B) Suspension of NPs of chitosan/TDP/TPP.
Figure 5: FT-IR spectrum corresponding to TPP matrix, NPs of chitosan/TPP and chitosan matrix, respectively.
Figure 6: FT-IR spectrum corresponding to TDP matrix, NPs of chitosan/TDP/TPP and chitosan matrix, respectively.
Figure 7: Graph of size evaluation in stability study in accelerated conditions (25°C ± 3°C) of chitosan NP suspensions.
Figure 8: Graph of polydispersion evaluation in stability study in accelerated conditions (25°C ± 3°C) of chitosan NP suspensions.
Figure 9: Graph of size evaluation in long-term stability study (5°C ± 3°C) of chitosan NP suspensions.
Figure 10: Graph of polydispersion evaluation in long-term stability study (5°C ± 3°C) of chitosan NP suspensions.
Figure 11: MTT of NPs of chitosan and chitosan/thiamine in comparison with the respective solutions.
Figure 12: Comparison of cell viability between NPs of chitosan/TPP and the chitosan solution at equal concentration.
Figure 13: Comparison of cell viability between NPs of chitosan/TDP/TPP and the chitosan/TDP solution at equal concentration.
Figure 14: Effect of the solution of chitosan, chitosan/TDP and chitosan NPs in a concentration equivalent to 50 μg/ml on the TEER of the monolayers of Caco-2 cells.
Figure 15: Effect of the solution of chitosan, chitosan/TDP and NPs of chitosan in a concentration equivalent to 100 μg/ml on the TEER of the monolayers of Caco-2 cells.

**DISCLOSURE OF THE INVENTION**

The technology corresponds to a chitosan-based nanoparticle system for the transport of peptides with activity in the central nervous system. This system would allow these peptides to reach the CNS with the objective of controlling the integrity of the BBB and creating a treatment alternative for a series of neurodegenerative diseases in which the neuroinflammatory cascade generates changes in the barrier.

The production process for this nanoparticle system allows the formation of chitosan NPs through the ionic gelation method using sodium tripolyphosphate (TPP) as a crossing-linking agent and thiamine pyrophosphate as a signal agent (TDP). The NP formation process included an analysis of the influence of different parameters, such as the chitosan/TPP/TDP mass relationship, pH, process temperature, time, speed, type of agitation and the incorporation of TDP into the developed NPs. At the same time, a comparison was made between the NPs obtained from chitosan/TPP and from chitosan/TPP/TDP.

The conditions for obtaining optimised **chitosan/TDP/TPP** NPs are described in Table 1.

The chitosan/TDP/TPP NPs produced with the optimum formulation had an average submicrometric diameter of 75,7 ± 2,5 nm, an irregular form, a smooth surface, and presented a positive zeta potential of 26,2 ± 0,45 mV, which allowed the NPs to be stable for a prolonged period of time, the formulation being highly reproducible. The NPs obtained present a stability at 5°C ± 3°C of 6 months without the incorporation of any stabilising agent in the medium, the samples not showing great variations with regard to size and polydispersion, in contrast to what is obtained at higher temperatures, where a stability of 7 days at 25°C ± 2°C with a relative humidity of 60% ±5% was observed. The optimum mass relationship between chitosan/TDP/TPP is 3:3:1, shown by the lower size of NPs with monomodal dispersion at 79,28 nm ± 2,5 nm, with a polydispersion index of 0,131 ± 0,025.

**Table 1:** Optimum conditions for obtaining NPs from chitosan/TDP/TPP.

| Condition | Range |
| --- | --- |
| Agitation | Ultraturrax® |
| Agitation speed | 8000 ± 1 rpm |
| Total agitation time | 1 - 3 minutes |
| Process temperature | 25 ± 1°C |

(continued)

| Condition | Range |
|---|---|
| Temperature of the solutions | 25 ± 1°C |
| Standard TPP concentration | 0,1 ± 0,01 % w/v |
| Standard chitosan concentration | 0,25 ± 0,01% w/v |
| Standard TDP concentration | 0,25 ± 0,01% w/v |
| TPP pH solution | 3,0± 0,1 |
| Chitosan/TDP pH solution | 4,5± 0,1 |
| Incorporation | TPP on chitosan |
| Mass relationship | 3:3:1 |

The mechanism proposed for the formation of de nanoparticles of chitosan-TPP considers that the ionic gelation of the chitosan occurs through electrostatic interactions between products from the dissociation of the TPP in an aqueous solution with the $NH3^+$ chitosan groups in an acid medium. The proposed production mechanism involves a high-speed preparation process that makes it possible to obtain NPs smaller in size than 100 nm.

The fact that the TDP is internalised in the NPs of chitosan/TDP/TPP and the pH of the suspensions is close to 5.0 implies that the microenvironment to which the cells in contact with the NPs are exposed is more innocuous, non-toxic and does not affect the viability of these cells.

The NPs are capable of introducing a modification in the tight junctions that are produced when the cell monolayer is completely formed and furthermore it was possible to determine that this modification in the tight junctions is reversible, with a recovery rate of 93% in the case of the NP solutions of chitosan/TDP/TPP. This recovery is dependent on the concentration used: the higher the concentration of the NPs, the lower the percentage of recovery.

The proposed NPs allow the encapsulation of different molecules or peptides of pharmacological interest, for which the molecules are incorporated into the solution of chitosan/TDP of 25% w/v, where the molecule load corresponds to 5 % of the total mass of the NPs. The solutions were left to react during magnetic agitation at 200 rpm for 2 hours, after which the NPs were prepared and characterised using dynamic light scattering analysis.

The NPs loaded with the active molecules have a hydrodynamic diameter of 80 ± 6 nm, with a Z potential of 26,1 ± 0,97 mV, and a polydispersity that is not greater than 0,150 PDI.

This method of encapsulation has an encapsulation efficiency of (EE%) 31,24 ± 2,8 and an active substance load percentage (Load %) of 0,65 ± 0,03.

The size of the NPs conditions their load capacity, which can be modified drastically if the size of the NPs is increased. With the same protocol, NPs of 250 nm ± 10,5nm in size were obtained, which resulted in a load % equivalent to 4,38 % ± 1,8.

The ideal is to have NPs smaller in size than 100 nm, if intravenous administration is desired; however, with a size under 300 the NPs can be administered intranasally without a problem, thereby increasing the load percentage of the molecules in the NPs, so that the choice of size depends on the preferred administration route.

## EXAMPLES OF APPLICATION

### Example 1: Obtention of chitosan/TPP NPs

The method for obtaining NPs from optimised **chitosan/TPP** is described as follows:

- Agitation: Ultraturrax®
- Agitation speed: 8000 rpm
- Total agitation time: 2 minutes
- Process temperature: 25°C ± 1°C
- Temperature of the solutions: 25°C ± 1 °C
- Standard TPP concentration: 0,1 % w/v
- Standard chitosan concentration: 0,25 % w/v
- TPP pH solution: 3,0
- Chitosan pH solution: 5,5
- Incorporation: TPP on chitosan

- Mass relationship: 3:1

**Example 2: Optimisation of the process to obtain NPs from chitosan/TDP/TPP.**

**2.1 Mass relationship between chitosan/TDP/TPP**

It was decided to test the direct incorporation of TDP into the NP formulation. For this purpose, different solutions were prepared containing a fixed concentration of chitosan 0,25% w/v and variable concentrations of TDP, generating different mass relationships between the matrix components. 4,0 ml of a 0,1% w/v TPP solution were incorporated directly into these solutions. The NP suspensions were developed according to the parameters established after the multiple response analysis.

The NPs produced at the beginning from chitosan/TDP/TPP were prepared following the modifications carried out by Rojanarata *et al,* according to which the TDP was dissolved previously and subsequently the chitosan was dissolved in the same solution.

After the mixing of the chitosan/TDP and TPP solutions a transparent suspension was observed. When the resulting suspensions were subjected to both a TEM and dynamic light scattering analysis, irregularly formed NPs were identified with a size that oscillated between 79 and 86 nm. For the purposes of studies *in vivo* the NPs below 100 nm can be used for BBB transport studies.

Table 2: Summary of size and polydispersity of the NP suspensions obtained, in different mass proportions.

| Mass relationship Chitosan/TDP/TPP | Size (nm) | Polydispersity |
|---|---|---|
| 3:3:1 | 79,28 | 0,131 |
| 3:3,6:1 | 80,64 | 0,157 |
| 3:4,2:1* | 85,58 | 0,132 |

* The sample analysis detected the presence of a polymer dispersed in the suspension

The dynamic light scattering analysis and the analysis of the NP suspensions observed in Table 2 showed the optimum mass relationship between the chitosan/TDP/TPP, which was 3:3:1, evidenced in the lower size of NPs with monomodal dispersion to 79,28 nm, with a polydispersity index of 0,131.

**2.2 pH of the chitosan/TDP solution**

Once the optimum mass relationship for the new formulation of NPs of chitosan/TDP and TPP was established, a pH study of the chitosan/TDP solution was conducted in order to obtain NPs smaller than 80 nm. For this purpose, 4 pH values were studied that were adjusted once the solution that contained TDP had been prepared, in the first instance, and once this was dissolved the chitosan was incorporated into the same solution. The resulting pH without adjustment was 2,6. With this solution NPs with a size close to 120 nm were obtained. Table 3 shows the values obtained after the dynamic light scattering analysis.

Table 3: Summary of the size and polydispersity of the NP suspensions obtained with different pH levels.

| pH Solution Chitosan/TDP | Size (nm) | Polydispersity |
|---|---|---|
| 4,0 | 86,45 | 0,135 |
| 4,5 | 72,42 | 0,147 |
| 5,0 | 90,10 | 0,146 |
| 5,5 | 103,2 | 0,174 |

It was decided to work with a chitosan/TDP solution that had a pH level of 4,5, since this pH made it possible to obtain NPs with a size of 72,42 nm. This value allows us to use these NPs for the transport of drugs through the BBB.

**2.3 Determination of optimum agitation speed in the formulation of NPs of chitosan/TDP/TPP.**

With the aim of optimising the size of NPs of chitosan/TDP/TPP, it was decided to re-evaluate the agitation speed used for the production of the NPs, maintaining the values of the fixed parameters analysed previously. For this purpose, NPs were developed through high-speed agitation with the Ultraturrax® method. Trials were carried out using 3 speeds varying between 8000 and 13500 rpm. The resulting NP suspensions were submitted to a dynamic light scattering analysis in which their size and polydispersity were examined.

**Table 4:** Evaluation of the agitation speed for NPs of chitosan/TDP/TPP.

| Agitation speed (rpm) | Size (nm) | Polydispersity |
|---|---|---|
| 8000 | 72,42 | 0,147 |
| 9500 | 87,72 | 0,160 |
| 13500 | 83,86 | 0,130 |

Table 4 shows a minimum effect in relation to the average diameter of the NPs when the agitation speed is changed during the production process. It was therefore decided to establish a speed of 8000 rpm as a production parameter, which resulted in NPs of chitosan/TDP/TPP that were irregular in form and had a smooth surface. This agitation speed led to smaller sizes, with less energy input into the system, thereby aiding suspension stability.

**2.4 Determination of the TDP inclusion percentage in the formulation**

The quantity of TDP included directly in the formulation was determined by using UV spectrophotometry to quantify the supernatant of the NP suspension. To do this, the NP suspensions were transferred to centrifugation tubes and were centrifuged for 25 minutes at 19000 at 15 °C. Subsequently, a T DP calibration curve ($\lambda$w= 246,9 nm) was done. The concentrations of the standards varied between 1x10-5 and 5x10-5 M (pH of 4,5). The solutions of the supernatants from the centrifugation were diluted and the presence of TDP was quantified.

The TDP determination gave an inclusion percentage of 78% $\pm$ 4,8 according to the analyses performed for the indirect determination of this in the NP suspension. The interaction that is produced between the TDP and the TPP is mediated by the formation of ionic pairs that form between the positive charges of the chitosan and the negative charges of the TDP.

**Example 3: Characterisation of the NPs of chitosan/TDP/TPP.**

The NPs were characterised according to their size, shape, size distribution, infrared spectroscopy and Zeta potential. The NPs of chitosan/TPP and chitosan/TDP/TPP were analysed in order to compare their characteristics.

**3.1 Transmission electron microscopy (TEM)**

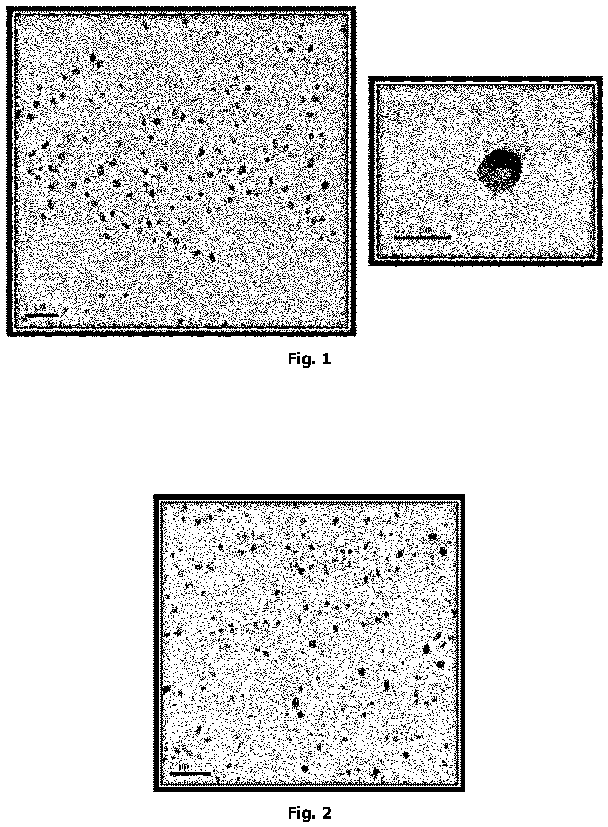

The nanoparticles were observed by depositing a drop of suspension liquid filtered through a 0,45 $\mu$m nylon filter on a copper grid covered by a carbon film left to dry at ambient temperature for approximately 12 hours. The presence of microparticles, nanoparticles and dispersed polymer in the suspension were evaluated. In both suspensions of NPs obtained through optimisation, only NPs without the presence of dispersed polymer were found, as can be seen in Figure 1, NPs of chitosan/TPP, and in Figure 2, NPs of chitosan/TDP/TPP.

**3.2 Dynamic light scattering**

This dynamic light scattering analysis was conducted with Zetasizer ZS90 equipment to determine size (average diameter), size distribution and quantity of particles in the Np suspension. The suspensions were irradiated using a laser and as a result of the brownian motion of the particles and the laser dispersion the equipment could interpret these signals as the sizes of the NPs present. The samples were deposited in a polystyrene cuvette and analysed in triplicate at a temperature of 25°C and at an angle of 90°.

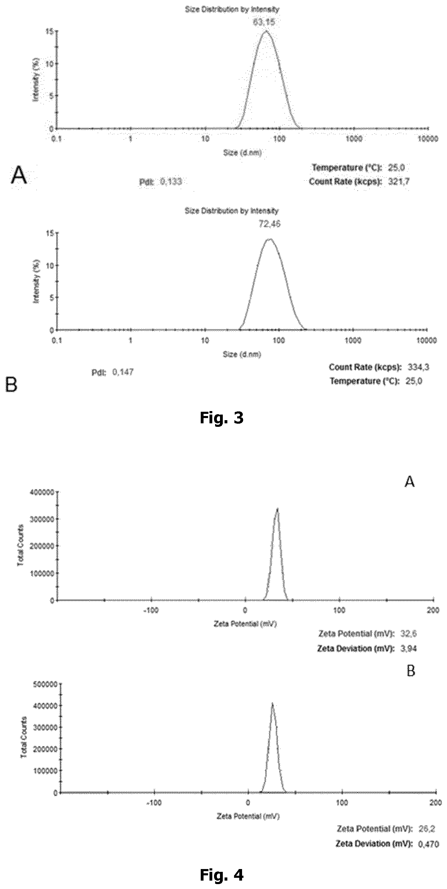

The dynamic light scattering analysis of the NPs of chitosan/TPP and of chitosan/TDP/TPP after the optimisation of the production process showed the presence of NPs in both suspensions with an average diameter of 64,5 $\pm$ 1,8 nm and 75,7 $\pm$ 2,5 nm, respectively (Figure 3), with unimodal characteristics, without the presence of polymers dispersed or added to the formulation. The obtention of NPs with sizes smaller than 100 nm in both final formulations supports subsequent studies of the internalisation of the NPs in the brain, since a uniform size smaller than 100 nm is needed for NPs to be internalised in the brain and thus cross the BBB.

**3.3 Zeta potential.**

The zeta potential of the NPs was studied to verify the stability of the suspensions. The analysis was conducted with Zetasizer ZS90 equipment, the principle of this form of measurement being the application of an electric field to a cell, in this case a special polycarbonate cuvette that possesses conducting cells. The zeta potential measurement is carried out using laser Doppler electrophoresis. The suspension samples were measured directly and diluted in a proportion of 10:1, from the conducting cells. Each analysis was conducted in triplicate at 25 °C. A zeta potential of 32,6 $\pm$3,9 mV was identified in the NPs of chitosan/TPP and of 26,2 $\pm$ 0,45 mV in the NPs of chitosan/TDP/TPP. This quality allows the NPs to be stable for a prolonged period of time. This analysis served to verify the position of the components of the matrix in the NPs. Furthermore, a positive charge indicated the external position of the chitosan with respect to the TPP in the NPs. Another property that can be inferred from the analysis is the adherence of the NPs to the tissues. Figure 4 shows the analysis conducted with Zetasizer equipment on both NP suspensions.

### 3.4 Fourier transform infrared spectroscopy (FTIR)

For the FTIR spectroscopy, all the components of the matrix of the NPs were analysed separately (chitosan, TDP and TPP). A small quantity of each was placed in an agate mortar with KBr excess. The mixture was triturated and finally put in a container to make an analysis tablet. The suspensions of NPs of chitosan/TPP and chitosan/TDP/TPP were freeze-dried for 24 hours and the resulting solid was pulverised and subjected to the same process previously described in order to obtain the NP spectrum and make a comparison between the individual components of the matrix and the respective NPs.

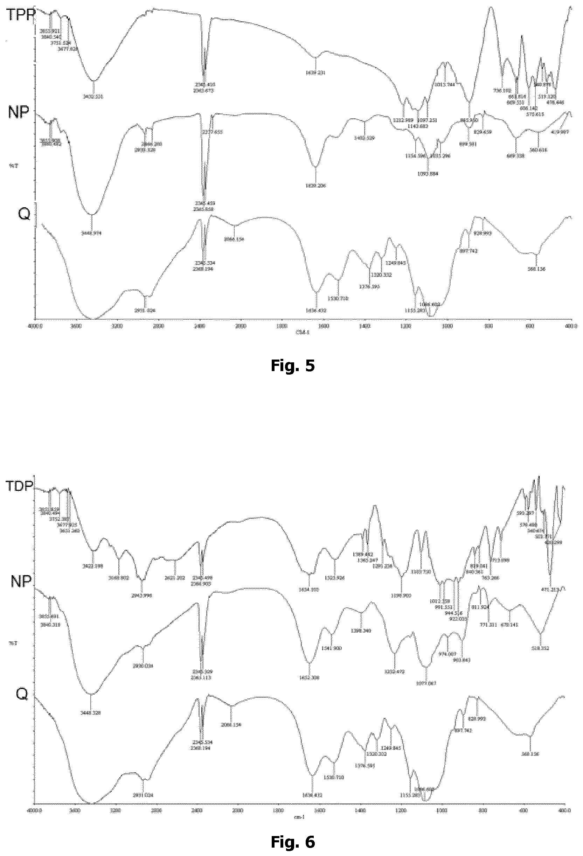

From the FTIR spectra analysed, the band localised at 1320 cm$^{-1}$ was taken as the characteristic band and the band at 1420 cm$^{-1}$ was used as a reference. FTIR spectroscopies of the freeze-dried NPs of chitosan/TPP (Figure 5) and chitosan/TDP/TPP (Figure 6) were performed. When the chitosan spectrum in the FTIR spectrum was analysed, three characteristic peaks were observed: at 3365 cm$^{-1}$ corresponding to the band (OH), at 1153 cm$^{-1}$ for the band (COC) and at 1579 cm$^{-1}$ for the band ($NH_2$).

The spectrum of the NPs of chitosan/TPP was different from that of the chitosan matrix. In the case of the NPs of chitosan/TPP, the vibration corresponding to the $NH_2$ flection at 1579 cm$^{-1}$ diminishes; on the other hand, two new peaks appear at 1638 cm$^{-1}$ and 1700 cm$^{-1}$, probably due to the protonation of the $NH_2$ groups that now are $NH_3$ ions and to the formation of the hydrogen bond between the O-TPP and the H of the free amino groups.

An analysis of the chitosan spectrum shows a band at 3482 cm$^{-1}$, indicating the presence of the OH group (stretching vibration). A change was observed in the NPs of chitosan/TDP/TPP from 3482 cm$^{-1}$ to 3449 cm$^{-1}$, a reduction in the width of the band being noted, which indicates the reduction in the hydrogen bonds. The reduced quantity of hydrogen bonds in the complexes of reticulated NPs is due to the more open structure that results from the reticulation with the TPP and TDP present in the matrix.

Another alteration in the FTIR spectra that indicates the formation of NPs is the change in bands 1657 and 1576 cm$^{-1}$ to 1643 and 1536 cm$^{-1}$ in the NP spectrum, which represents the $CONH_2$ and $NH_2$ groups, respectively. The displacement of these two bands and the increased intensity of the $NH_2$ band were also observed by other researchers, which suggests ionic interaction between the chitosan amino groups charged positively and the TPP phosphate groups charged negatively.

### 3.5 Stability study for NPs of chitosan/TPP and of chitosan/TDP/TPP

For the stability study of chitosan NP suspension, it was decided to use the methodology given in both the ICH Q-1A norm and the stability guide established by the Public Health Institute of Chile (ISP in Spanish) for pharmaceutical forms destined to be conserved through refrigeration. The accelerated, long-term working conditions can be seen in Table 5. The analysis of the samples includes the data from 3 production batches and the statistical evaluation of quantitative data that may change over time. It is established with a unilateral confidence limit of 95% for the average acceptance criterion.

The following changes were evaluated as significant in the stability parameters:

- A 5% reduction in the valuation of the measurement parameter (size, polydispersity and quantity of particles) compared with zero time.
- Presence of degradation products.
- Non-fulfilment of acceptance criteria because of changes in appearance.

**Table 5:** Stability study conditions for the NPs of chitosan/TPP and chitosan/TDP/TPP.

| Study | Storage conditions | Analysis time | Evaluation frequency |
|---|---|---|---|
| **Real time** | 5°C $\pm$ 3°C | 6 months | 1,15, 45 days and then monthly |

(continued)

| Study | Storage conditions | Analysis time | Evaluation frequency |
|---|---|---|---|
| **Accelerated time** | 25°C ± 2°C 60% HR ± 5% HR | 30 days | First 3 days and then every 2 days |

The study involved the preparation of a suitable quantity of NP suspension in triplicate of chitosan/TPP and chitosan/TDP/TPP. An aliquot of 4 ml of the respective suspensions was stored in penicillin bottles and sealed under pressure, each one of these bottles corresponding to a sample in a determined period of time. A fourth sample was stored, consisting of a 1:1 dilution of the respective suspensions of NPs with NaCl 0.9 % w/v. The samples were subjected to dynamic light scattering analysis, in which their size and polydispersity were examined and the results were analysed using a two way ANOVA.

One of the most important properties of a pharmaceutical formulation is related to the formulation stability, especially in the case of chitosan NPs, since characteristics such as the suspension size in solution become critical when suspensions are stored for long periods of time.

In the case of suspensions stored at 25°C (see Fig ures 7 and 8) the study had to be stopped after the samples had been stored for 7 days because of the presence of fungi in the formulation, which greatly changed the aqueous medium of the formulations of both NPs of chitosan/TPP and NPs of chitosan/TDP/TPP. This was mainly due to the fact that the pre-established storage conditions of raw materials such as chitosan (Protasan®) is under refrigeration. If these criteria are not fulfilled, as in the case of storage at 25°C, the NP suspension medium i s destabilised by the degradation of the chitosan NPs, thereby modifying the aqueous medium and facilitating the proliferation of microorganisms such as fungi and bacteria. The samples became visibly turbid and produced sediments because of the presence of fungi and perhaps because of the substantial swelling of the chitosan NPs caused by their storage at 25°C, as turbidity is associated with an increase i n the size of the particles in the medium due to the entry of water into the NPs by osmosis, due to the presence of TPP. As a result, the NPs expand and the chitosan forming the matrix of the NPs fractures.

The statistical analysis sought to establish if both formulations had the same effect on the size of the NPs in relation to all the times analysed, which was discounted with a p value > 0,05, since the type of formulation does not directly influence the variation in size if each time is evaluated. At the same time, the research tried to determine which of the two formulations had a greater effect on the variability in size of the nanoparticles, but the effect was considered not to be significant with a p value of > a 0,05. Thus, one formulation does not prevail over the other. Finally, the effect of time on each formulation was investigated and it was determined that time indeed effects the formulations but not very relevantly, the p value being 0,0528.

The polydispersity analysis showed that the type of formulation and the storage time have an extremely significant effect with a p value of < 0,05 with regard to the influence on the variability presented by the two formulations in relation to polydispersity.

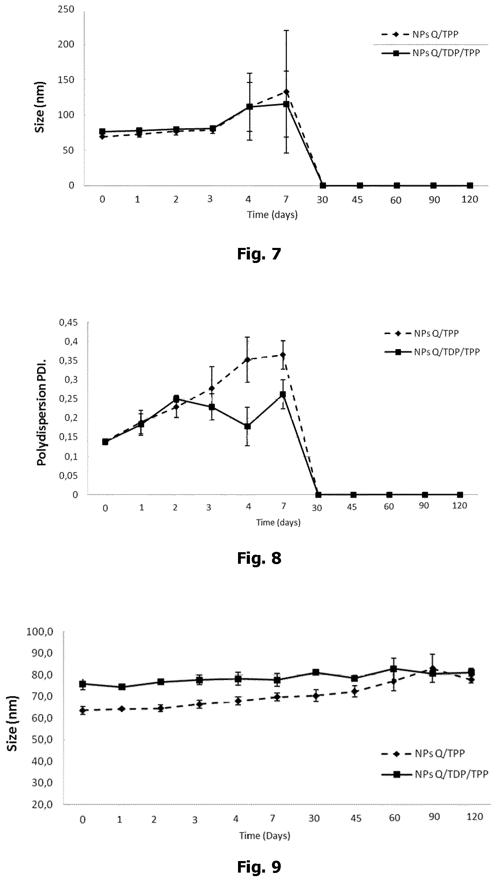

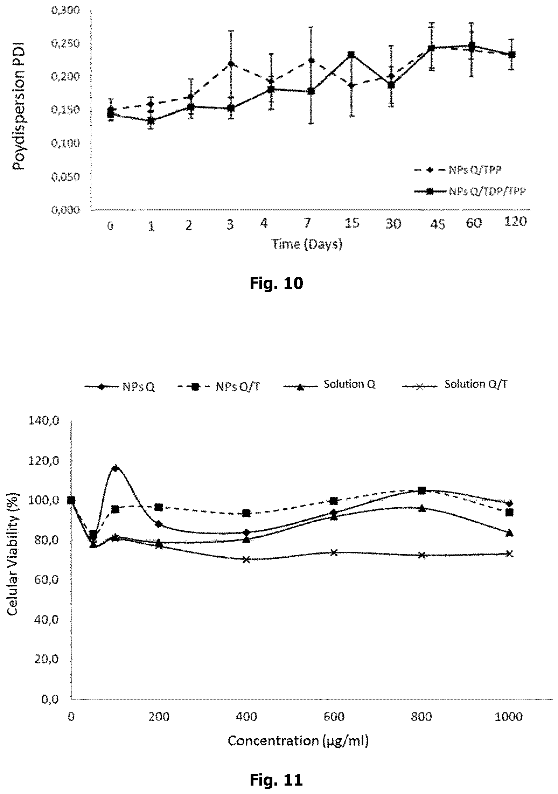

The study of the suspensions stored at 5°C was 100% completed, covering 120 days. The samples did not suffer great variations in size and polydispersity (see Figures 9 and 10), largely because the pre-established storage conditions for the raw materials (Protasan®) were respected. The statistical analysis sought to establish if the two formulations had the same effect on the size of the NPs in relation to all the times analysed, if the formulations had any influence on the variation in size of the NPs and, finally, if time affected the formulations. The results showed that each parameter evaluated statistically was highly significant with regard to the variations in size in the formulations of NPs with a p value of < 0,05.

The polydispersity analysis showed that the type of formulation and the storage time do not have a direct effect on the polydispersity of the chitosan NP suspensions.

**Example 5: Cell viability study**

The Caco-2 cells were used for the determination of all the studies that included an epithelial model. They were obtained after 7 days of incubation at 37°C with 5% $CO_2$ with a DMEM culture medium supplemented by 10% BFS. The culture medium was changed once a day during the 7 days of incubation. The results obtained were reproducible every time that the cell line with a 97.5% average viability was needed.

**5.1. Preparation of reagents for MTT test** 3-(4,5-dimethylthiazol-2-yl)-2,5-diphenyltetrazolium bromide

The MTT test was used for the cell viability study. This method is simple in that cell viability is given by the number of cells present in the culture, which can be measured through the formation of a coloured compound, due to a reaction that takes place in the mitochondria of viable cells. The MTT (3-(4,5-dimethylthiazol-2-yl)-2,5-diphenyltetrazolium bromide) is captured by the cells and reduced by the enzyme mitochondrial succinate dehydrogenase to its insoluble form formazan. The product of the reaction is retained in the cells and can be released by solubilisation with SDS (sodium lauryl sulphate).

## 5.2 Preparation of cells and seeding of culture plates

48-well plates and Caco-2 cells with 7 days of confluence with a cell viability greater than 95% were used for the MTT test. The cells were placed in the 48-well plates in a quantity equivalent to 200,000 cells/well and were then left in culture chambers at 37°C with 5% $CO_2$ for 24 hours. After this time an MTT test was performed using the following protocol:

a) Complete elimination of the 48-well plate culture medium.
b) Placing of NPs with culture medium in concentrations equivalent to 50 -100-200-400-600-800 and 1000 $\mu$g/ml of chitosan.
c) An exposure time of the cells with NPs of 3 hours at 37°C with 5% $CO_2$.
d) After 3 hours the DMEM culture medium with 10% BFS that contained the NPs was eliminated and the cells were washed with sterile PBSI; then 100 $\mu$l of RPMI medium (free of red phenol) were incorporated; finally 10 $\mu$l of MTT reagent were added and the plates were left to culture at 37°C with 5% $CO_2$ for 2 hours.
e) After these 2 hours, 100 $\mu$l of SDS were added to each well and the plates were left to culture for a maximum period of 16 hours in order to dissolve the formazan crystals that were produced by the reaction of the MTT.
f) The plates were subjected to spectrophotometrical analysis at a wave length that fluctuated between 540 nm and 570 nm and the stimulation index (SI) was calculated to determine the % of cell viability, taking into consideration the SIs corresponding to each sample x and the $SI_0$ corresponding to the Caco-2 cells without exposure to external agents.

$$SI = \frac{Sample\ Absorbance - Blank\ Absorbance}{Blank\ Absorbance}$$

## Equation 1: Calculation of stimulation index

$$Cell\ viability = \frac{SIx}{SIo} \text{ x 100}$$

## Equation 2: Calculation of cell viability %

The analyses were octuplicated and the effect on the cell viability of the following external agents was evaluated:

1-Chitosan solution
2-Chitosan/TDP solution
3-NPs of chitosan/TPP
4-NPs of chitosan/TPP/TDP

In addition, the effect of the respective controls was evaluated, these being a control of DMEM medium with 10% BFS free of external agents, a control of solvent equivalent to a solution of NaCl 50 mM, and a positive control consisting of highly concentrated SDS.

The cell viability determination showed that the Caco-2 cells exposed to chitosan NPs have a cytotoxicity lower than 20%, as can be seen in Figure 11. However, the chitosan solutions cause greater damage to cells in comparison with chitosan NPs.

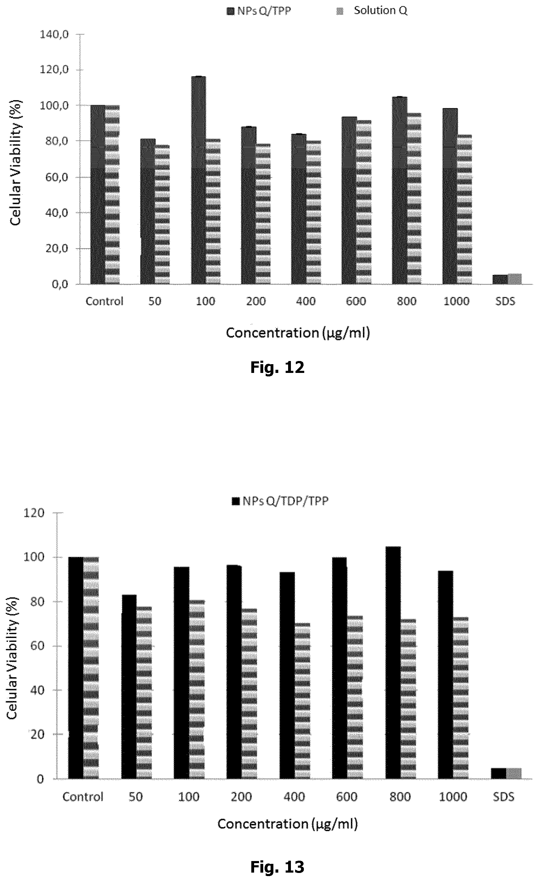

Figure 12 shows in greater detail the difference between the NPs of chitosan/TPP and the solution of chitosan at the same concentrations, a reduction existing in the average cell viability of not higher than 20% in all the concentrations of chitosan analysed with the exception of the concentration of 100 $\mu$g/ml. This may be partly explained by a possible seeding of a greater number of cells in that plate well, consequently generating less deterioration. On the other hand, the control, which corresponded to the saline medium in which chitosan NPs were inserted, did not produce any damage, so that the effect on cell viability is 100% attributable to the NPs of chitosan/TPP or to the chitosan solution. If a comparison is made between the effect on cell viability of the NPs of chitosan/TPP and that of the chitosan solution, it can be seen that the chitosan solution creates less damage at the same concentration than the NPs of chitosan/TPP. This could be because TPP is present in the NPs of chitosan/TPP, TPP being a molecule that can also affect the cell viability of Caco-2 cells when they are exposed to it directly.

Figure 13 shows in greater detail the difference between the NPs of chitosan/TDP/TPP and the solution of chitosan/TDP at the same concentrations, a reduction existing in the average cell viability not higher than 15% in the case of the NPs of chitosan/TDP/TPP in all the concentrations of chitosan analysed, in relation to the solution of chitosan/TDP in which can be seen a greater effect on cell viability depending on the concentration, since at higher chitosan concentrations (400 -600 - 800 and 1000 $\mu$g/ml) cell damage bordering 30% is observed. This may be partly explained by the fact that, in the case of the NPs of chitosan/TDP/TPP, the TDP is internalised in the NPs and the pH of the suspensions is close to 5,0, while in the case of the suspension of chitosan/TDP the TDP exists freely in the solution and the pH is 4.5. This means that the pH of the external agent to which the Caco-2 cells are exposed should be considered an important factor in cell damage, which would explain the great difference that occurs when Caco-2 cells are exposed either to the NPs of chitosan/TDP/TPP or to the chitosan/TDP solution.

**Example 6. Determination of transepithelial resistance (TEER)**

**6.1 Determination of the variability in the TEER in the presence of NPs of chitosan/TPP and NPs of chitosan/TDP/TPP**

The TEER determination used Transwell plates with 24 wells with a membrane diameter of 0,33 cm$^2$. The plates were seeded with a volume equivalent to 200000 cells/well after the cells had had 7 days of confluence. The TEER was measured daily until it reached a resistance value that appeared to be constant (300 $\Omega$). Once this value had been reached, the culture medium was removed from the plate and was replaced by a suspension of NPs of chitosan/TPP and NPs of chitosan/TDP/TPP in a DMEM culture medium with 10% BFS and the TEER values were evaluated at 0-0,5-1-1,5-2-4-6-8 and 10 hrs. After the exposure to the NPs, the culture medium was eliminated, the cells were washed with sterile PBS and placed again in a DMEM culture medium with 10% BFS, free of external agents. The TEER was again evaluated, this time 12 and 24 hours after the culture medium was replaced. The chitosan NPs were evaluated in addition to the respective solutions of chitosan, TPP and TDP, accompanied by a control with a DMEM medium with 10% BFS free of external agents and a control of solvent equivalent to a solution of NaCl 50 mM. Concentrations of 50 $\mu$g/ml and 100 $\mu$g/ml of chitosan were used for all the analyses, which were carried out in triplicate and the results were analysed using one-way ANOVA.

$$TEER = (R\ Monolayer - R\ Blank)\ x\ Membrane\ Area$$

**Equation 3:** Calculation of TEER

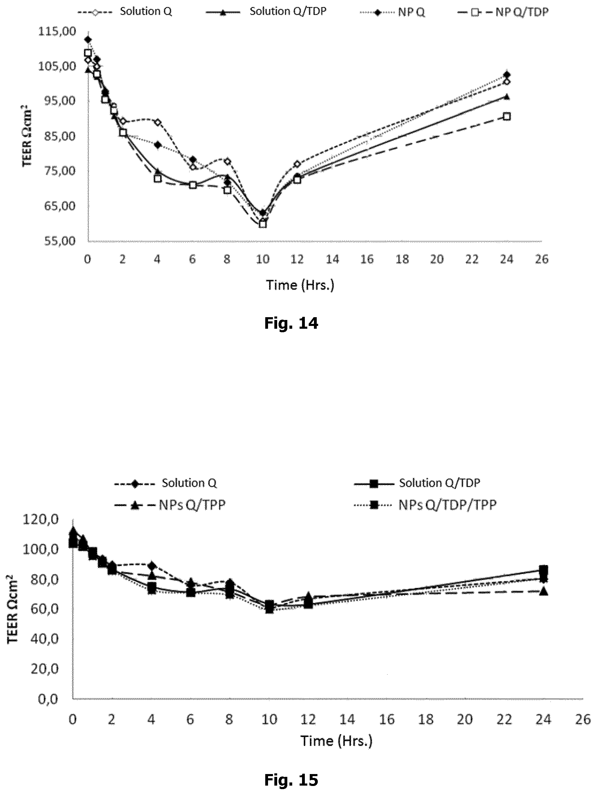

The TEER variation in Caco-2 cell monolayers was monitored in order to study the effect of chitosan in solution and in the form of NPs of chitosan/TPP or NPs of chitosan/TDP/TPP on the integrity of the tight junctions. Figure 14 shows that both the NPs and the solution of chitosan and chitosan/TDP caused a significant reduction (p<0,05) in the TEER of the monolayers with respect to the initial TEER and with respect to the control. Additionally, the effect of the chitosan solution on the TEER was significantly less than that of the nanoparticles (p<0,05).

The fall in TEER was observed both with the solutions of chitosan and chitosan/TDP as with the NPs of CH/TDP/TPP, although the effect was more pronounced in the case of the NPs. This may be due to the positive surface charge effect, higher in the NPs, which aids the mucoadhesion of the NPs on cell membranes. The greater the number of positive charges the stronger the interaction with the negative charges of the cell membranes. Therefore this may mean that the effect on the opening of the tight junctions is more prominent.

An analysis of the TEER values obtained once the NPs are exposed confirms the fact that the chitosan NPs are capable of modifying the tight joints produced when the monolayer of the Caco-2 cells is completely formed. In addition, it was discovered that this modification in the tight joints is reversible, 94% and 93% of membrane integrity being recovered in the case of the solutions of chitosan and chitosan/TDP; and 90% and 93% in the case of the NPs of CH/TPP and NPs of CH/TDP/TPP, respectively. When the movement of ions across the monolayer is restricted because the tight joint functions correctly, there exists an electric potential gradient on both sides. For this reason, the integrity and maturity of the tight junctions is normally determined by measuring the transepithelial electric resistance (TEER), which is inversely proportional to the permeability. In this case it was possible to establish that the chitosan is able to alter the permeability reversibly, thereby aiding the transport of different substances across different epithelia.

With the objective of establishing if the effect of the solutions of chitosan, chitosan/TP and of the NPs of CH/TPP and CH/TDP/TPP was dependent on the concentration of chitosan, the effect of the solutions at a higher concentration (100 $\mu$g/ml) on a Caco-2 cell monolayer was evaluated. Figure 15 shows that the recovery of the TEER is dependent on the chitosan concentration to which the Caco-2 cell monolayer is exposed, since at higher concentrations, although the same TEER reduction effect is obtained, after the cells have been exposed to the chitosan solutions and NPs the recovery of the monolayer is lower, with a membrane integrity recovery percentage of about 70%.

**Example 7: Encapsulation of TNF-α blocking molecule (E1: Etanercept).**

To encapsulate the molecule, an aliquot of previously purified E1 was incorporated into a solution of chitosan 0,25% w/v and a solution of chitosan/TDP 0,25% w/v equivalent to 5% of the solid mass load present in the NPs produced. The solutions were left to react with magnetic agitation at 200 rpm for 2 hours. After this time, 4 mL of TPP solution 0,1% w/v were incorporated directly. The NPs were prepared and characterised using dynamic light scattering analysis.

To determine the quantity of E1 encapsulated in the NPs the BCA method was used and the process to obtain the E1 was carried out according to the following protocol:

a) Centrifuge 500 μl of the sample (chitosan NPs with encapsulated E1) at 14000 rpm for 20 min at a temperature of 20°C using Amico n® Ultra-0,5 centrifugation filters.
b) Remove immediately the supernatant retained in the filter holder and transfer the filter to an inverted filter holder to receive the pellet formed.
c) Centrifuge the filter at 1000 rpm for 2 min to obtain the retained pellet.
d) Add 1 mL of acetic acid 0,2 N to the pellet.
e) Agitate in vortex for 5 min.
f) Sonicate for 10 min.
g) Dilute as necessary to obtain a concentration within the calibration curve range for the BCA method.

The quantity of encapsulated E1 was expressed with the parameters of the encapsulation efficiency percentage (EE %) and the load percentage of the active substance (load %), which is expressed in Equations 4 and 5.

$$EE \% = \frac{Encapsulated\ E1\ mass}{Total\ E1\ mass} \times 10$$

**Equation 4:** Calculation of the encapsulation efficiency percentage

$$Load \% = \frac{Encapsulated\ E1\ mass}{Total\ polymer\ mass} \times 100$$

**Equation 5:** Calculation of the E1 load percentage

It was possible to encapsulate E1 in NPs of chitosan/TPP and NPs of chitosan/TDP/TPP. The resulting NPs reached a hydrodynamic diameter of 73,2 ± 2,5 nm and 83,1 ± 4,5 nm respectively, with a Z potential of 30,1 ± 0,58 mV and 26,01 ± 0,97 mV. In the case of both formulations the polydispersion did not exceed 0,150 PDI. Table 6 shows the levels of encapsulation efficiency (EE %) and of load % reached after the process of encapsulation of BSA in the NPs. It should be pointed out that the mass balance equals 101 and 105 % respectively.

**Table** 6: Results of BSA encapsulation in NPs of CH/TPP and NPs of CH/TDP/TPP.

| Formulation | EE% | Load % |
|---|---|---|
| NPs of CH/TPP | 35,67 ± 2,4 | 0,61 ± 0,02 |
| NPs of CH/TDP/TPP | 31,24 ± 2,8 | 0,65 ± 0,03 |

Although it was possible to encapsulate E1, the encapsulation load % was lower than 1%. This was due principally to the fact that the size of the chitosan NPs used was smaller than 100 nm, which greatly influences the load capacity of the chitosan NPs. This value can be modified drastically if the size of the chitosan NPs is increased. Using the same protocol to obtain the chitosan/TPP NPs, it was decided to increase their size to 250 nm ± 10,5nm, which produced a load % equivalent to 4,38 % ± 1,8, thereby demonstrating that size is the most important factor limiting the E1 load of NPs.

**Example 8: Identification of the presence of NPs in the CNS.**

**8.1 Incorporation of the fluorescent marker.**

First, 250 mg of chitosan were dissolved in 25 ml of nanopure water and 10 mg of fluorescein isothiocyanate (FiTC) were dissolved in 1 mL of ethanol. The two solutions were mixed with permanent magnetic agitation for 24 hours in a dark room with a temperature of 37°C. The resulting conjugate was purified by dialysis, for 72 hours, (using dialysis tubes with a pore size of 12 KDa) and the medium was changed every 24 hours. The isolated solution of chitosan-FiTC was frozen at -55°C in an ethanol bath, and subsequently freeze-dried to completely extract the residual solvent.

**8.2 Preparation of fluorescent NPs coated in polysorbate 80.**

Due to the surface characteristics that polysorbate 80 offers NPs with respect to their incorporation into the CNS, it was included as a coating agent for NPs to be administered intravenously. For this purpose, the chitosan-FiTC was dissolved in nanopure water and its pH was adjusted to 4.5. The TPP solution was mixed with the polysorbate 80 (1% v/v) for 24 hours and then the NPs were prepared, as described in Examples 1 and 2.

The suspensions obtained were subjected to dynamic light scattering analysis to establish the average diameter (Table 7).

**Table 7:** Size distribution of NPs marked with FiTC.

| NP composition | Average diameter ± DS [nm]. | PDI |
|---|---|---|
| Chitosan/TPP/ without polysorbate 80 | 131 ± 13 | 0,231 ± 0,108 |
| Chitosan/TPP/with polysorbate 80 | 75 ± 11 | 0,463 ± 0,125 |

The results showed NPs of FiTC whose average diameters fluctuated between 75 and 130 nm, with or without polysorbate 80, respectively. These NPs were used to conduct the following tests to verify their transfer to the CNS.

**8.3 *In vivo* tests.**

Once the NPs were characterised, *in vivo* tests were conducted in order to verify the transfer of the NPs across the BBB. These tests were carried out according to the protocol relating to the use of animals at the University of Concepción. For this purpose, 5 male and female Sprague Dawley rats weighing between 250 and 450 grammes were used. The suspension of marked NPs was administered intravenously (IV). Tables 8 and 9 summarise the conditions of the study:

**Table 8:** Formulations administered in the *in vivo* study.

| Formulation | NP composition | Polysorbate 80 coating |
|---|---|---|
| N°1 | NPs-FiTC | No |
| N°2 | NPs-FiTC | Yes |
| Control | NaCl 0,9 % w/v. | No |

**Table 9:** *In vivo* study

| Rat N° | Volume administered* [µL] | Formulation administered | Administration route | Sacrifice time [min.] |
|---|---|---|---|---|
| 1 - 2 | 500 | 1st | IV | 60 |
| 3 - 4 | 300 | 2nd | IV | 60 |
| 5 | 300 | Control | IV | 60 |

*The volumes injected were calculated according to the weight of each rat. The concentration of the suspension was below the LD50 of all the formulation components.

After the animals were sacrificed, their brains, liver and spleen were extracted and placed in a fixing solution composed of 4% paraformaldehyde and phosphate buffer saline (PBS) 1 M. Subsequently these were cut up to improve the penetration of the fixer and kept for 24 hours at ambient temperature.

The histological sections of the different organs were made with a vibratome. The brain sections had a thickness of 50-60 $\mu$m, the liver sections a thickness of 40 $\mu$m and the spleen 30 $\mu$m. The brain sections were stained with Dapi by immersion for 30 minutes (1/1000 in PBS). Subsequently the sections were observed using confocal microscopy and spectral confocal microscopy.

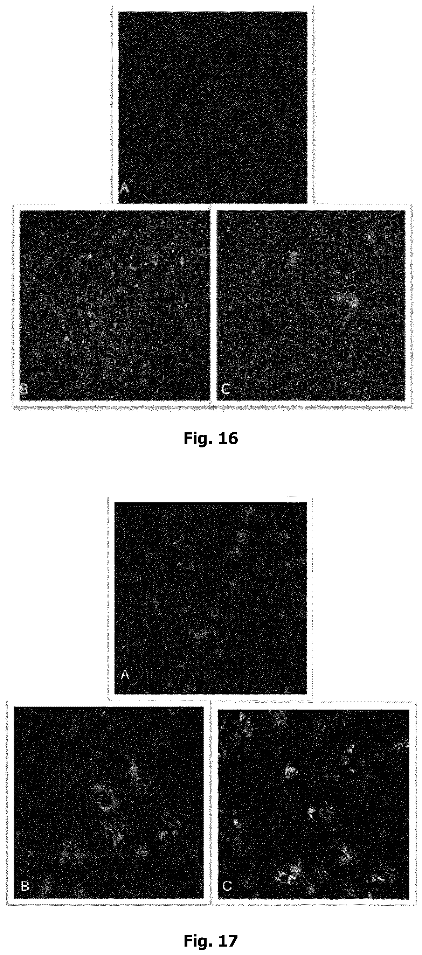

The histological analysis of the control rat liver showed little fluorescence (Fig. 16A), unlike that from the rats that received NP suspension N°1 (Fig. 16B) or NP suspension N°2 (Fig. 16C), where the NPs can be see n inside the hepatocytes, in addition to greater fluorescence in this organ's vessels. The arrival of the formulation in the liver shows that the latter is part of the reticular system and the purifier of substances that enter the blood stream. This also proves the efficacy of the coating in aiding the NPs to bypass this organ belonging to the reticular system.

The analysis of rat spleens, (Fig. 17), also an organ belonging to the reticular system, showed high basal fluorescence in the tissue (A) in the histological sections made. This increased when the suspensions of fluorescent NPs N°1 and N°2 were injected (B and C respectively). Given that the rat spleen analysis without the incorporation of NPs showed basal fluorescence due to the presence of metabolisation components, it is not possible to assert with 100% certainty that the presence of fluorescence in this organ is attributable to the presence of NPs, making it difficult to interpret the analysis of the spleen in particular.

The rat brain analysis showed little green fluorescence near the hippocampal neuronal nuclei, after administering the control (A). However, in (B), corresponding to the hippocampus of the rats injected with NPs coated with polysorbate 80, it was observed that the NPs were present in the brain microcirculation vessel and in nearby areas. Fluorescent points were also found near to neuronal nuclei (arrows). The brain specificity mechanism of the NPs coated with polysorbate 80 is not totally known, but it is believed that polysorbate 80-coated NPs bind with the apolipoprotein E present in the plasma. This binding may be responsible for the orientation of the NPs in the brain and may lead them to imitate low-density lipoproteins (LDL), and thus force the LDL receptors present in the brain vascular endothelial cells to recognise them as such and trigger their absorption, through a endocytosis mechanism followed by transcytosis across the endothelium and the subsequent reuptake by the neurons in the cerebral parenchyma. As the size of the NPs controls the endocytosis rate across the endothelial cells of the brain capillaries, because their size is similar to that of LDLs, associated with the presence of polysorbate 80 on the surface of the NPs, this triggers the recognition of the specific receptors, thereby aiding their internalisation across the BBB.

## Claims

1. Chitosan-based nanoparticles for the transport of peptides with activity in the central nervous system CHARACTERISED because composed of:

   a. chitosan, as a base;
   b. sodium tripolyphosphate (TPP), as a cross-linking agent; and
   c. thiamine pyrophosphate (TDP), as a signal agent.

2. Chitosan-based nanoparticles, according to Claim 1, CHARACTERISED because the chitosan/TDP/TPP are in a mass relationship of 3:3:1.

3. Chitosan-based nanoparticles, according to Claim 1, CHARACTERISED because they possess an average diameter of 80 $\pm$ 6nm.

4. Chitosan-based nanoparticles, according to Claim 1, CHARACTERISED because they possess an average diameter of 250 $\pm$ 10,5nm

5. Chitosan-based nanoparticles, according to Claim 1, CHARACTERISED because they present a positive zeta potential of 26,2 $\pm$ 0,45 mV.

6. Chitosan-based nanoparticles, according to Claim 1, CHARACTERISED because they present a stability at 5°C $\pm$ 3°C of 6 months without the incorporation of any stabilising agent in the medium.

7. Chitosan-based nanoparticles, according to Claim 1, CHARACTERISED because they present a polydispersity index of 0,131 $\pm$ 0,02.

8. Chitosan-based nanoparticles, according to Claim 1, CHARACTERISED because they present an encapsulation efficiency of 31,24 $\pm$ 2,8.

9. Chitosan-based nanoparticles, according to Claim 1, CHARACTERISED because they present an active substance load percentage of 0,65 $\pm$ 0,03%.

10. Chitosan-based nanoparticles, according to Claims 1 and 4, CHARACTERISED because they present an active substance load percentage of 4,38% $\pm$ 1,8%.

11. Use of chitosan-based nanoparticles, according to Claim 1, CHARACTERISED because they are used to produce a drug useful for treating diseases of the central nervous system.

12. Use of chitosan-based nanoparticles, according to Claims 11 and 9, CHARACTERISED because they are used to produce a drug that is administered intravenously.

13. Use of chitosan-based nanoparticles, according to Claims 11 and 10, CHARACTERISED because they are used to produce a drug that is administered intranasally.

**Fig. 1**

**Fig. 2**

Size Distribution by Intensity
63,15

A

Pdl: 0,133

Temperature (°C): 25,0
Count Rate (kcps): 321,7

Size Distribution by Intensity
72,46

B

Pdl: 0,147

Count Rate (kcps): 334,3
Temperature (°C): 25,0

**Fig. 3**

A

Zeta Potential (mV): 32,6
Zeta Deviation (mV): 3,94

B

Zeta Potential (mV): 26,2
Zeta Deviation (mV): 0,470

**Fig. 4**

**Fig. 5**

**Fig. 6**

**Fig. 7**

**Fig. 8**

**Fig. 9**

**Fig. 10**

**Fig. 11**

**Fig. 12**

**Fig. 13**

**Fig. 14**

**Fig. 15**

**Fig. 16**

**Fig. 17**

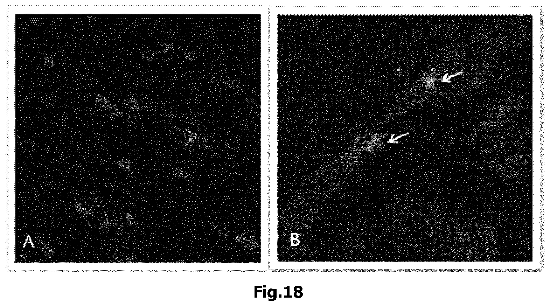

**Fig.18**

## INTERNATIONAL SEARCH REPORT

| | International application No. |
| --- | --- |
| | PCT/ CL2015/000067 |

**A.    CLASSIFICATION OF SUBJECT MATTER**

(CIP) A61K 9/51, 31/722, 31/51, C08B 37/08, C08L 5/08 (2016.01)

According to International Patent Classification (IPC) or to both national classification and IPC

**B.    FIELDS SEARCHED**

Minimum documentation searched (classification system followed by classification symbols)

(CIP) A61K 9/51, 31/722, 31/51, C08B 37/08, C08L 5/08

Documentation searched other than minimum documentation to the extent that such documents are included in the fields searched

Electronic data base consulted during the international search (name of data base and, where practicable, search terms used)

Thomson, Google Patents, Pubmed, Pubchem,

**C.    DOCUMENTS CONSIDERED TO BE RELEVANT**

| Category* | Citation of document, with indication, where appropriate, of the relevant passages | Relevant to claim No. |
| --- | --- | --- |
| Y | CASTILLO, E. et al. Development of a nanoparticle carrier as transport strategy through the nervous system. Journal of Pharmacology Chile. August 2014; 7(2): 17-24. ISSN 0718-882X [the whole document] | 1-13 |
| Y | LOCKMAN, P. R. et al. Brain uptake of thiamine-coated nanoparticles. Journal of Controlled Release. 2003; 93: 271-282. DOI: 10.1016/j.jconrel.2003.08.006 [pag 276-281] | 1-13 |
| Y | TIWARI, S. B. et al. A review of nanocarrier-based CNS delivery systems. Current Drug Delivery. 2006; 3 (2): 219-232. DOI: 10.2174/156720106776359230 [pag. 226-227 Table 3] | 1-13 |
| Y | FAZIL, M. et al. Development and evaluation of rivastigmine loaded chitosan nanoparticles for brain targeting. European Journal of Pharmaceutical Sciences, abril 2012; 47: 6-15. DOI: 10.1016/j.ejps.2012.04.013 [pag. 9- Table 4 and 5] | 1-13 |

| [X]  Further documents are listed in the continuation of Box C. | [ ]  See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 03/03/2016             3 March 2016 | 06/04/2016           6 April 2016 |

| Name and mailing address of the ISA/ | Authorized officer |
| --- | --- |
| INAPI, Av. Libertador Bernardo O'Higgins 194, Piso 17, Santiago, Chile | CARTIER UGARTE, Denise |
| Facsimile No. | Telephone No.   56-2-28870551   56-2-28870550 |

Form PCT/ISA/210 (second sheet) (January 2015)

## INTERNATIONAL SEARCH REPORT

International application No.

PCT/ CL2015/000067

C (Continuation). DOCUMENTS CONSIDERED TO BE RELEVANT

| Category* | Citation of document, with indication, where appropriate, of the relevant passages | Relevant to claim No. |
|---|---|---|
| Y | HAQUE, S. et al. Venlafaxine loaded chitosan NPs for brain targeting: Pharmacokinetic and pharmacodynamic evaluation. Carbohydrate Polymers, feb 28 2012; 89: 72-79. DOI: 10.1016/j.carbpol.2012.02.051 [pag. 73, 75 and Table 3 and 4] | 1-13 |
| A | ROJANARATA, T. et al. Chitosan-thiamine pyroposphate as a novel carrier for siRNA delivery. Pharmaceutical Research. 2008; 25(12): 2807-2814. DOI: 10.1007/S11095-008-9648-6 [the whole document] | |
| A | MD, S. et al. Bromocriptine loaded chitosan nanoparticles intended for direct nose to brain delivery: Pharmacodynamic, pharmacokinetic and scintigraphy study in mice model. European Journal of Pharmaceutical Sciences. Die 2013; 48: 393-405. DOI: 10.1016/j.ejps.2012.12.007 [the whole document] | |
| A | FERNANDEZ-URRUSUNO, R. et al. Enhancement of nasal absorption of insulin using chitosan nanoparticles. Pharmaceutical Research. 1999; 16(10): 1576-1581 [the whole document] | |

Form PCT/ISA/210 (continuation of second sheet) (January 2015)